# EUROPEAN PATENT APPLICATION

(11) **EP 2 944 249 A1**
(43) Date of publication of application: **18.11.2015**
(21) Application number: 14706969.4
(22) Date of filing: 10.01.2014
(51) Int. Cl.: A61B 1/267, A61B 1/00

(54) **DIGITAL LARINGOSCOPE**

(30) Priority: 10.01.2013 PT 10673013
(71) Applicant: Universidade do Porto, 4099-002 Porto (PT)
(72) Inventor: RAMOS DA SILVA, António José, 4475-617 Maia (PT); MAGALHÃES MENDES, Joaquim Gabriel, P-4200-465 Porto (PT); RODRIGUES QUINTAS, Manuel, P-4050-465 Porto (PT); DE PINHO E COSTA AMORIM, Pedro, 4099-003 Porto (PT); NATAL JORGE, Renato Manuel, P-4200-465 Porto (PT)
(74) Representative: Pereira da Cruz, Joao
(86) International application number: PCT/PT2014/000002
(87) International publication number: WO 2014/109659

(57) **Abstract**

The present invention discloses a laryngoscope to help endotracheal intubations with the ability to measure and record the torque resulting from the force applied on the standard laryngoscope blade commercial available.

The laryngoscope is composed by a standard blade (18) equipped with an optical fiber and is articulated in the pin (17) located in the laryngoscope head (41); the main body (42), were are all the electronic components and sensors are installed being closed by the handle cover (43). The laryngoscope has wireless communication capability and can communicate with a remote computer or other portable devices where a designed application is used to monitoring, visualization and recording all acquired data. The device has an inertial unit (24) to determine its spatial orientation according to the three main axis. The sound or/and visual alarms (13, 1) correspondent to an eventual excessive force compared to a certain predefined level are available in the laryngoscope handle head (41) or in the remote0 computer or mobile device. The laryngoscope includes also a LED to illuminate oral cavity, with variable intensity according to the user needs.

The sensors in this Laryngoscope make it suitable for hospital use, in the training of professionals in dummies or other situation where the opening of the oral cavity is required. Allowing this way the visualization of the movement and torque in real time, as well as recording and afterwards reviewing the procedure.

## Description

### Technical Domain of the Invention

The present invention discloses a laryngoscope used to perform throat exams and support the endotracheal intubations. The laryngoscope has wireless communication capability and can communicate with remote computer or other portable devices where a designed application is used to monitoring, visualization and recording all acquired data. This Laryngoscope is suitable for hospital use, in the training of professionals in dummies or other situation where the opening of the oral cavity is required. Allowing this way the visualization of the movement and torque in real time, as well as recording and afterwards reviewing the procedure.

### State of art

From the state of the art, it is already known devices able to perform endotracheal intubations with force measuring capabilities, however on these devices the force, or pressure, is directly measured in the blade using piezoelectric or piezoresistive films that limit its usage since the blade has to be sterilized after every usage, which may damage or even destroy the sensor and adds an extra a contamination or injury risk for the patient.

One of these patents is the EP1433413A2, which reveals a laryngoscope blade covered with a polymeric piezoresistive material. In this case the blade is not reusable, making therefore an increased cost in each use.

In the patent US5070859 it is presented a pressure measurement laryngoscope, using in this case piezoelectric sensors, applied directly in the blade. On the other hand, the patent EP0793440B1 describes a laryngoscope capable of measuring the contact force with the upper teeth of the patient, once again using a piezoelectric film.

Finally, the patent document WO2009130666A1 presents a Laryngoscope with sensors in the handle. This device uses capacitive sensors to detect the presence of the user hand when this grabs the handle. When the hand is detected, the illumination is tuned on. This way the sensors serve only to switch the light not to measure force or torque.

### Summary of the invention

The goal of the current invention is to describe a device to support the endotracheal intubations procedure composed by:
- a handle where the sensing modules are installed, like torque, spatial orientation, wireless communication, power and battery charger, embedded Computational unit based in microcontrollers and all the supporting elements for the standard blade, preferable made of stainless steel with the specific fit and the optical fiber for the light conduction;
- a computational application running on a computer or in any other mobile device, capable of communicate with the laryngoscope.

The blade (18) is articulated in the pin (17) transmitting the force applied to the blade through the pin (19) properly guided and hosted in a Low friction sleeve element (21) that transmits the applied force to the force sensing element (10) positioned in the interior of the handle head (41) with two supporting elements one at each side (9, 11). This way the standard blade does not require any covering or electrical connections. Additionally, the dynamic response of this system compared with the piezoresistive sensors is far superior, allowing the perception of any quick variations of the applied force.

The laryngoscope handle includes not only the force sensors but the inertial system (accelerometer, gyroscope, inclinometer and/or compass) connected to an embedded computational unit based on a microcontroller and connected to the wireless bidirectional communication system. This way, it is possible to know in real time the spatial orientation of the laryngoscope and its movement, which is potentially useful in the trainee of the health professionals, since it is possible to monitor the performance for future analyses. The laryngoscope also includes a warning system, visual and/or audible with threshold conditions previously by the user. During the device usage the warning signals respond according with data acquired by the sensing systems, being activated by any sensor, or any combination of them. It is also possible to disable this functionality.

All the electric and electronic systems are located inside the handle and are supplied by a rechargeable battery. The illumination is set according with the user specification through a high power LED and conducted by an optical fiber located in the in the blade, all the way to the blade tip.

### Brief figures description

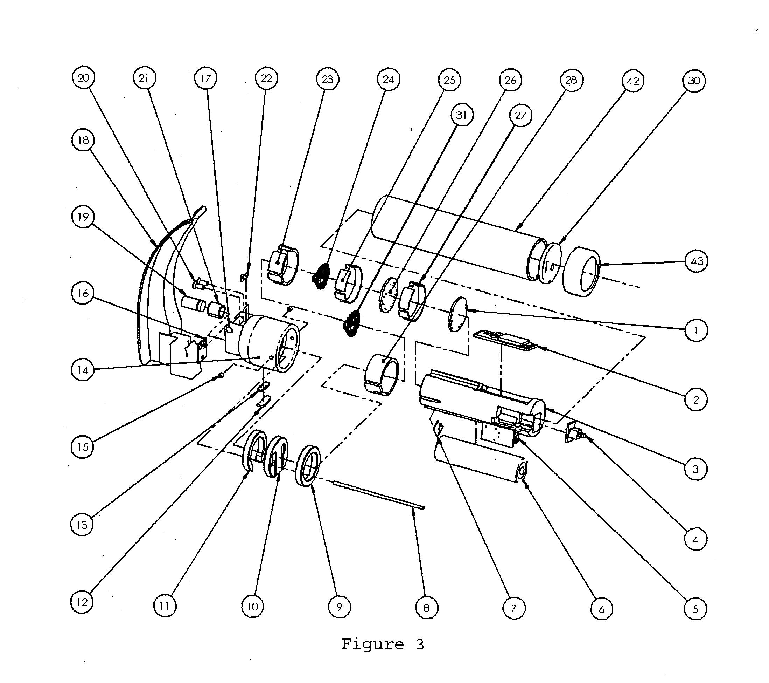

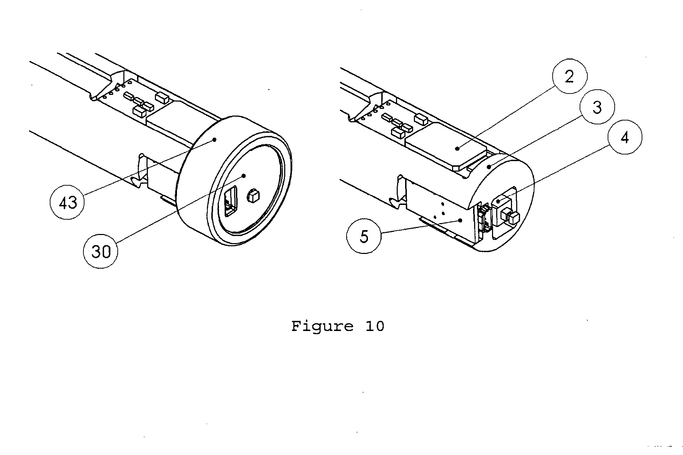

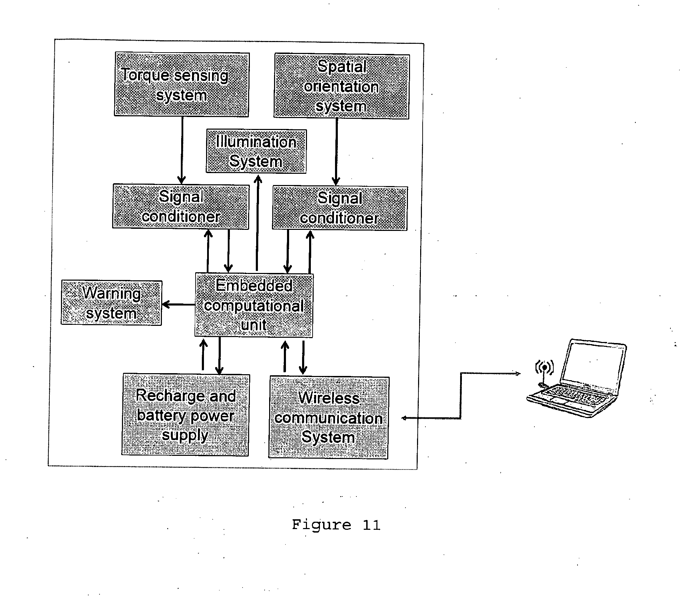

To a more direct understanding of the invention, there are attached some figures, which represent preferable designs of the invention that do not limit the present invention.

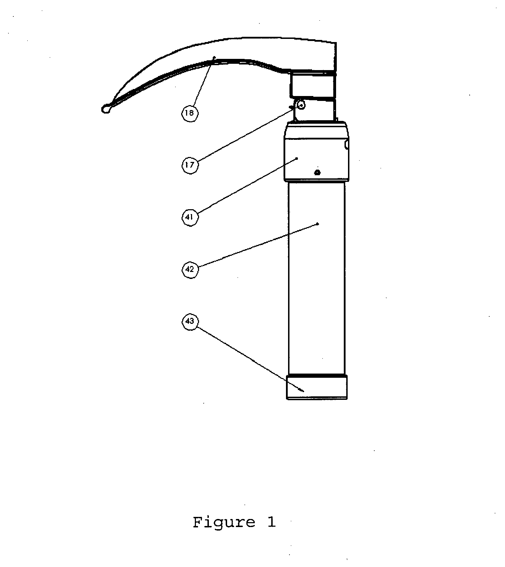

**Figure 1****:** lateral view of the laryngoscope described in the invention. The blade is articulated in the pin 17, as in the state of the art.
   17 - Blade articulation pin
   18 - Blade
   41 - Handle head
   42 - Handle main body
   43 - Handle cover.

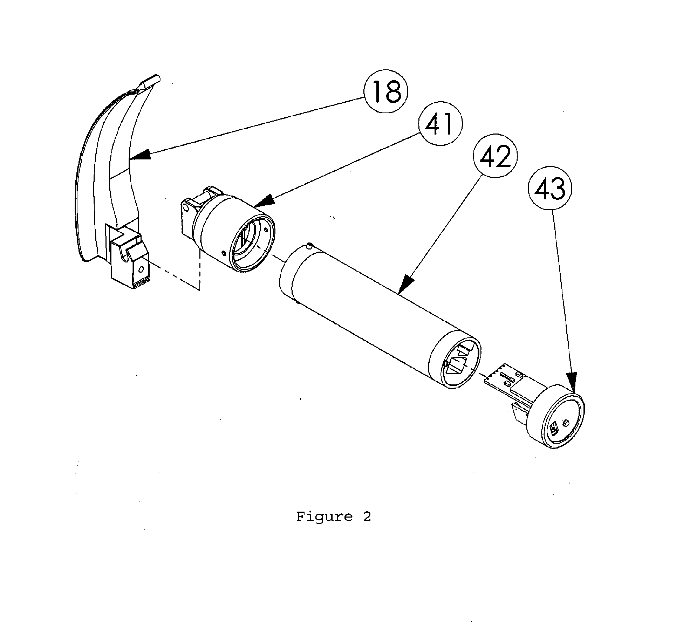

**Figure 2****:** Isometric view of the laryngoscope as described in this invention. The handle is composed by the handle head (41) were are allocated the force sensor elements, the main body (42) that includes the electronics and is closed by a flexible cover that overwraps the antenna, the on/off switch and the recharging connection, like for example a miniUSB.
   18 - Blade
   41 - Handle head
   42 - Handle main body
   43 - Handle cover
**Figure 3****:** Exploded view of the digital laryngoscope, illustrating the components composing one of the several configurations.
   1 - Sound alarm;
   2 - Wireless bidirectional communication module;
   3 - Housing inferior element;
   4 - Laryngoscope state switch;
   5 - Recharge and battery power supply;
   6 - Battery;
   7 - Battery contact and positioning flexible element;
   8 - Positioning and alignment element;
   9 - Sensor lower supporting element;
   10 - Force sensing element;
   11 - Sensor upper supporting element;
   12 - Visual warnings protection;
   13 - Visual warnings;
   14 - Main head element;
   15 - Fixation pin;
   16 - Illumination positioning and fixation element;
   17 - Blade articulation pin
   18 - Blade
   19 - Force transmission element;
   20 - Illumination system fixation screw;
   21 - Low friction sleeve element;
   22 - Variable illumination element;
   23 - Housing and positioning element of the inertial system;
   24 - Signal conditioner;
   25 - Alignment and positioning element of the electronic signal conditioning and microcontroller;
   26 - Electronic conditioning signal and microcontroller system;
   27 - Alignment and positioning element of the sound alarm;
   28 - Alignment and positioning element of the inertial system;
   30 - Handle base;
   41 - Handle head;
   42 - Handle main body;
   43 - Handle cover.

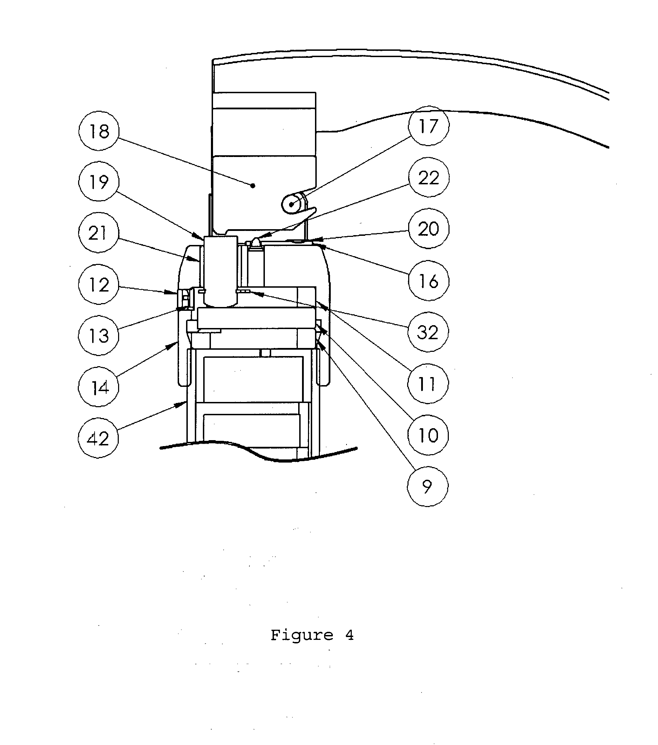

**Figure 4****:** Section view of the handle head (41) illustrating the force sensor (10) instrumented with electric resistance strain gages and supported by two elements with a circular shape (9, 11). The blade (18) is articulated in the pin (17) and supported by the force transmitting element (19) guided by the Low friction sleeve element (21) and is kept in position by the retaining ring (32). The visual warnings (12, 13) are also visible, the illumination LED (22) and all the elements inside the head main element (14).
   9 - Sensor lower supporting element;
   10 - Force sensing element;
   11 - Sensor upper supporting element;
   12 - Visual warnings protection;
   13 - Visual warnings;
   14 - Main head element;
   16 - Illumination positioning and fixation element;
   17 - Blade articulation pin
   18 - Blade
   19 - Force transmission element;
   20 - Illumination system fixation screw;
   21 - Low friction sleeve element;
   22 - Variable illumination element;
   32 - Retaining ring;
   42 - Handle main body;

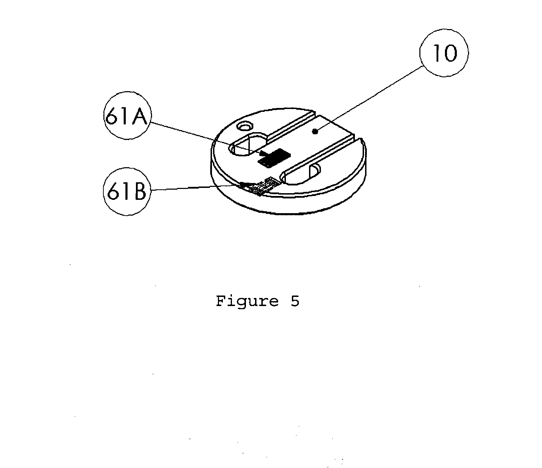

**Figure 5****:** Detail of the force sensing element (10) and respective strain gauges (61A and 61B).
   10 - Force sensing element;
   61A - Upper strain gauge;
   61B - Lower strain gauge;

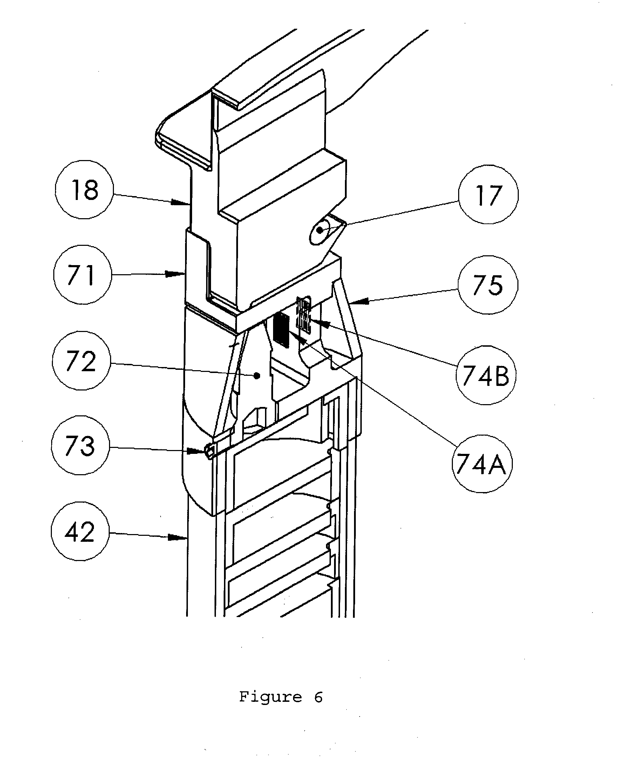

**Figure 6****:** Isometric section view of the force and torque sensing system. The main head of the system (41) displays in this figure a different configuration and design, integrating here the a double sensor element (71), being located at a different distance from the articulation pin (17), while the respective strain gauges (74A, 74B) measure the force and the torque.
   It is also visible the torque limit regulator (72), as well as the protection element of the sensing element (75). The configuration of the main head is also linked with the handle main body (42) like in the other configurations. This design has the advantage of measuring not only the torque, like the in the other configurations, but also the force location.
   17 - Blade articulation pin
   18 - Blade
   42 - Handle main body
   71 - Double sensing element;
   72 - Torque limit regulator;
   73 - Screw;
   74A - Strain gauge;
   74B - Strain gauge;
   75 - Sensing protection element;

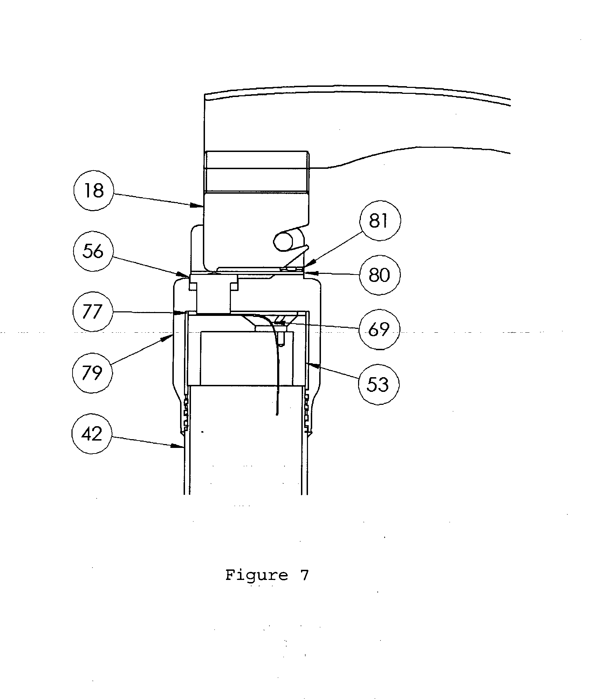

**Figure 7****:** section view of an alternative design of the device, based on piezoresistive sensors
   18 - Blade;
   42 - Handle main body;
   53 - Supporting element of the piezoresistive sensor;
   56 - Force transmitting element;
   69 - Force sensor;
   77 - Spacing element of the force sensor;
   79 - Main head with alternative design B;
   80 - Fixing element of the force transmitting component;
   81 - Screw.

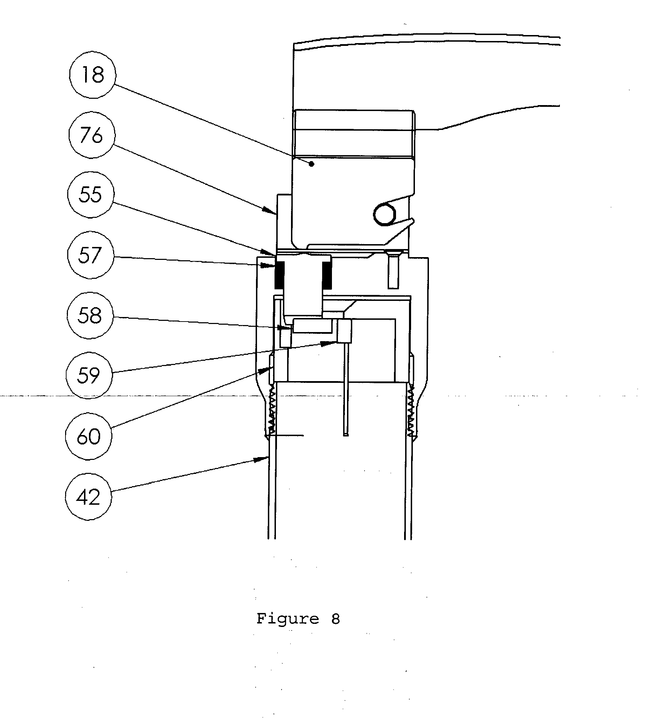

**Figure 8****:** section view of the device according with an alternative implementation based on magnetic sensing for torque measurement.
   18 - Blade
   42 - Handle main body
   55 - Displacement transmitting element;
   57 - Elastic element;
   58 - Magnetic element;
   59 - Magnetic sensor element;
   60 - Magnetic sensor supporting element;
   76 - Main head with alternative design A;

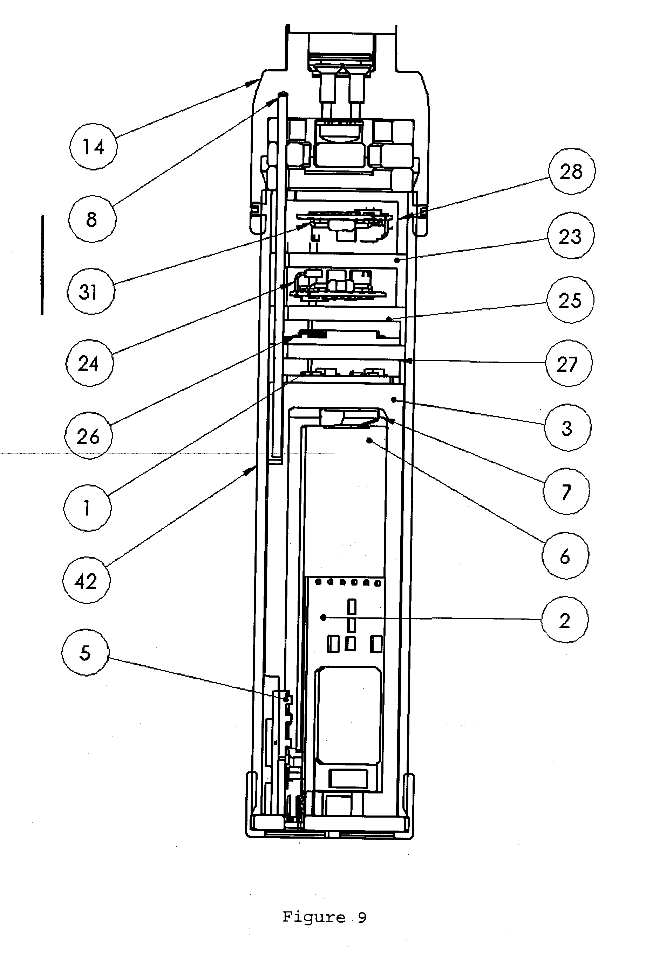

**Figure 9****:** section cut of the positioning pin, correspondent electronic systems and corresponding housing elements.
   1 - Sound alarm;
   2 - Wireless bidirectional communication module;
   3 - Housing inferior element;
   5 - Recharge and battery power supply;
   6 - Battery;
   7 - Battery contact and positioning flexible element;
   8 - Positioning and alignment element;
   14 - Main head element;
   23 - Housing and positioning element of the inertial system;
   24 - Signal conditioning;
   25 - Alignment and positioning element of the electronic
   26 - Electronic conditional signal and microcontroller system;
   27 - Alignment and positioning element of the sound alarm;
   28 - Alignment and positioning element of the inertial system;
   31 - Inertial system;
   42 - Handle main body
**Figure 10****:** Isometric section cut of the interior of the laryngoscope, illustrating the antenna (2) the plug of the battery recharging system (5), and the on/off state button (4).
   2 - Wireless bidirectional communication module;
   3 - Housing inferior element;
   4 - Laryngoscope state switch;
   5 - Recharge and battery power supply;
   30 - Handle base;
   43 - Handle cover
**Figure 11****:** scheme of the electronics. The electronic system is composed by a microcontroller, a wireless communication system, sound and visual warnings (LEDs), illumination LED, force sensors, inertial sensors, signal conditioning and a computer or other mobile device with wireless communication.

### Invention detailed description

A laryngoscope is a medical device usually used to assist endotracheal procedures and can originate several problems in the patient if not handled correctly, like: heart rate changes, blood pressure fluctuations, broken teeth, soft tissues injuries, etc.

The present device can be used in the intubation procedures or the training of the intubation procedure, using test dummies or other situations where it may be necessary the opening of the oral cavity.

The device disclosed in this invention allows the measuring of the torque applied blade and the resulting force from the contact with patient oral cavity organs such as the larynx, teeth, trachea etc. However, unlike the devices referred in the state of the art, this device has the measuring systems and mechanisms all inside the handle, releasing the blade from any sensor or electric cables, keeping the usage of standard blade available in the market and allowing the sterilization and following reuse without any damage in the sensors.

The laryngoscope is composed by two elements, the blade (18) and the handle (41, 42, 43), articulated in the pin (17), located in the top of the head (41) in a way that it allows the blade to close over it, reducing the laryngoscope occupied volume. The laryngoscope blade is usually curved to facilitate the intubation and can easily be removed to facilitate its exchange or sterilization. The handle is divided in three main parts, the head (41) the main body (42) and handle cover (43).

The head is composed by the articulation pin (17) and the main structural element (14) where are inserted the visual indicators (13) and respective protection (12). In the head exists also a force transmission element (19) and respective sleeve (21) alongside with the retaining ring (32), the force sensing element (10), correspondent lower and upper supports (9, 11) and the illumination system. The force sensing device is preferably materialized by a load cell like exemplified in figure 5 not excluding the other forms and designs. One of the possible implementations allows the measuring of force and torque. The main head has a configuration which integrates a double sensor element, each one of them maybe located at a different distance from the articulation pin (17), the strain gauges (74A and 74B) are used to perform the force and torque measurements. There is also an adjustable torque limitation (72) that guaranties the integrity of the sensors. Finally the sensing system protection element (75) closes the entire sensing mechanism inside of the main handle head. This configuration of the handle head maybe attached to the handle (42) like the previous described configurations. This arrangement has the advantage of allowing the measurement not only of the applied torque, like the others presented previously, but also the resulting pressure location.

The illumination system is constituted by the illumination with variable intensity (22), the positioning and fixation element (16) and the holding screws. By last, the handle head (41) is threaded and attached to the main body by fastening screws (15).

The main body allocates the electronics, the signal conditioning and supplying: power and battery recharging system (5), battery (6), power controller for the illumination system, microcontroller and part of the conditioning system (26), all of them allocated in the element (25), the inertial system (31), the microcontroller (26), the sound alarm system (1), the guiding pin (8) and the several positioning and housing elements (3, 23, 25, 27 and 28). The inertial system is capable of measuring the orientation and spatial movement of the laryngoscope, it may include accelerometers, compass, inclinometers and gyroscopes, in one, two or three main axis, according with the application requirements. The microcontroller is responsible to monitoring the signals from the sensing elements, interaction with wireless communication module, controlling the light intensity of the illumination system and the sound and visual warning signals. The different housing and positioning elements allocate the several electronic and electrical circuits, maintaining them in the correct position keeping their integrity during the assembly and the normal usage. However in the positioning system, the housing of the internal components (3) makes the connection between the main body (42) and the cover (43) also allocating the battery (6). The power supply, recharging systems and wireless communicating modules are partial inside the component (3).

The handle cover (43), covers the handle base (30) the recharge and battery power supply (5) the wireless bidirectional communication system (2) and the laryngoscope state switch (4). The handle cover is flexible allowing to switch the state of the laryngoscope, e.g. a switch (4) being also permeable to the wireless communication. The battery charging can be achieved through a common plug located in the base of the laryngoscope, protected by the handle cover (43) being this preferable a miniUSB port.

The torque measurement is accomplished by using a force measuring device, inside the head of the main body. In a preferable implementation, the actuation is achieved by a guided pin allowing the transmission of forces from the outside to the inside of the laryngoscope handle. This pin is supported by the force sensor, for example, by bending a flexible element instrumented with strain gauges. The application of a force in the blade causes the pin to move slightly, bending the flexible element and thus deforming the strain gauges. The strain gauges are connected to a signal conditioning system and this to a microcontroller allowing their measurement.

### Alternative design

In an alternative design, the force transmitter pin (56) can apply the force directly to a piezoelectric force sensor, preferable using low voltage or in a piezoresistive sensor, inserted in the main head (79).

Another alternative to the load cell, would be the usage of magnetic field sensors without contact, for example, Hall effect sensors or MGR. In this case, the element (55) would transport a small magnetic field generator (58), for example a magnet that by changing its distance from the magnetic sensor (59) would change the electric output of the sensor, enabling the monitoring of the applied force.

The laryngoscope includes an illumination LED with variable intensity controlled by the microcontroller, allowing a better adjustment to the environmental conditions, to the patient and to the health professional.

The laryngoscope still has visual and sound alarms, being the first for an example a RGB LED that changes its color with the variations of force. Alternatively, it can be used a VU meter to display the value of force in the form of a LED growing bar. The health professional can this way be warned if the values overcome certain predefined values. The sound alarm (1) is connected to the microcontroller that changes its amplitude, intensity or the tone, depending of the measured force,

The laryngoscope has the possibility of communicate wirelessly (bidirectional) with a remote station (for example a computer) through a special software. This way the computer allows the reproduction of sound or visual warnings, as a function of the measured values of force, inertial systems, as well as the setting of different alarm values.

The software allows the reception of the data from the laryngoscope through the wireless communication and represent them in real-time to the user. The user can define the units of the data, configure alarms, read signals, record, read and visualize the signals, or perform a detailed analyses of the performed movements, or previous force and torque, allowing this way general training of the use of the laryngoscopes.

## Claims

1. Digital laryngoscope with torque measurement capability and spatial orientation in real time, comprising:
a handle, composed by a main head (41), handle main body (42) and cover (43), preferably with a close cylinder shape, housing:
- an articulation pin (17) that is the connection between the blade and the main handle head(41);
- a force transition pin, with a custom design (19).
- a force sensing element (9, 10, 11);
- Signal conditioning electronic connected to the micro controller (26);
- Sound (1) and visual warnings (13) and all the blade supporting elements;
- a standard bade (18) with an optical fiber to conduct the light, articulated in the pin (17) allowing its rotation relatively to the handle;
where the torque and force measuring system are completely inside the handle;
and by having additionally:
- An inertial sensing system (24);
- A bidirectional communication system (2);
- A software application;
- A overall switch (4);
- A power supply and correspondent battery charging system (5);

2. Digital laryngoscope according with the claim 1, where the force sensing element (9, 10, 11) being a load cell type sensor.

3. A digital laryngoscope according with the claim 1, where the force sensing element being a double sensor (71) and each one can be positioned at a different distance from the articulation pin (17).

4. A digital laryngoscope according with the claim 1, where the blade (18) being articulated in the pin (17) that supports the force transmission pin (19) sliding, guided by a bushing (21).

5. A digital laryngoscope according with the claim 1 and 2, where the force transmission element (19) slides inside the bushing element.

6. A digital laryngoscope according with the claim 1, having a force sensor element composed by a sensor element (9,10,11) or a piezoelectric sensor of low voltage, a Hall effect sensor or a MGR.

7. A digital laryngoscope according with the claim 1, **characterized by** the inertial sensing system (24) is compose by acceleration sensors, compass, inclinometer and gyroscope according to one, two or three axis.

8. A digital laryngoscope according with the claim 1, **characterized by** the use of a controlled intensity LED in the illumination system (22).

9. A digital laryngoscope according with the claim 1, where the sound and visual indicators being located in the laryngoscope or/and in the computer.

10. A digital laryngoscope according with the claim 1, having the laryngoscope overall switch (4) protected by a cover (43) made of a flexible material.

11. A digital laryngoscope according with the claim 1, having the laryngoscope overall switch (4) protected by a cover (43) permeable to the wireless communications.

12. A digital laryngoscope according with the claim 1, integrating an application to perform data acquisition, monitoring, data analyses and processing and control of the laryngoscope.
